(19) Europäisches Patentamt
European Patent Office
Office européen des brevets

(11) Publication number: 0 008 831
B1

(12) EUROPEAN PATENT SPECIFICATION

(45) Date of publication of patent specification: 17.03.82

(51) Int. Cl.³: C 07 D 207/16

(21) Application number: 79200474.9

(22) Date of filing: 31.08.79

# (54) Process for the preparation of 1-(3-mercapto-2-D-methyl-propionyl)-L-proline and derivatives thereof.

(30) Priority: 07.09.78 NL 7809120

(43) Date of publication of application:
19.03.80 Bulletin 80/6

(45) Publication of the grant of the patent:
17.03.82 Bulletin 82/11

(84) Designated Contracting States:
BE DE FR GB IT NL

(56) References cited:
DE - A1 - 2 703 828

(73) Proprietor: Océ-Andeno B.V.
Grubbenvorsterweg 8
NL-5928 NX Venlo (NL)

(72) Inventor: de Heij, Nicolaas, Antonius
Wilgenstraat 22
Blerick (NL)

(74) Representative: Kremer, Robert A.M., Drs. et al,
Océ-Nederland B.V. Patents & Information
Postbus 101
NL-5900 MA Venlo (NL)

EP 0 008 831 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

Process for the preparation of 1-(3-mercapto-2-D-methylpropionyl)-L-proline and derivatives thereof

The invention relates to a process for the preparation of proline derivatives by reacting a proline compound with a derivative of 3-mercapto-2-methylpropionic acid, or β-mercapto-isobutyric acid.

Such a process is already known from the Dutch Patent Application, laid open for public inspection, No. 7701457. It describes, for example, the reaction of 3-acetylthio-2-methylpropionic acid chloride with L-proline to form 1-(3-acetylthio-2-DL-methylpropanoyl)-L-proline. Thus, the product is a mixture of diastereoisomers.

As compounds that may possibly be applied in the pharmaceutical field are concerned here, and the pharmaceutical effect is often coupled to only one of the stereoisomers — in the present case to the isomer showing the D-configuration in the β-mercaptoisobutyric group — the disadvantage attached to the above reaction is that the reaction product has still to be separated into optical isomers.

The object of the present invention is to provide a process in which this separation into optical antipodes can be dispensed with. This is achieved, and the process of the invention is characterized accordingly, by that there is started from an optically active D(—)-3-mercapto-2-methylpropionic acid of which the mercapto function is protected, to convert the acid into the corresponding acid chloride, which is then reacted with a L-proline compound according to Formula I of the formula sheet, in which R is a hydroxyl group, an amino group or a lower alkoxy group, and $R_1$ and $R_2$ represent independently a hydrogen atom, a hydroxyl group or a lower alkyl group, after which the protected mercapto function is liberated in order to obtain the desired proline derivative according to Formula II of the formula sheet, in which R, $R_1$ and $R_2$ have the meanings indicated above. Derivatives of the L-proline itself, so those wherein in the Formulae I and II of the formula sheet R=OH and $R_1$=$R_2$=H, are preferred.

The starting product D(—)-3-mercapto-2-methylpropionic acid (or D(—)-β-mercapto-isobutyric acid) of which the mercapto function is protected by means of one of the groups known for that purpose, is still a new compound; it can be prepared in a way as described in our Dutch Patent Application No. 7809121 bearing the same priority date as this application. Briefly that preparation comes to the following:

(1) there is started from a racemic mixture of 3-benzoylthio-2-methylpropionic acid,
(2) a suitable resolving agent, such as cinchonidine, is added,
(3) the cinchonidine D(—)-salt formed is crystallized out and isolated,
(4) if necessary, this salt is further purified from the L-isomer by recrystallization,
(5) the purified cinchonidine D(—)-salt is hydrolysed, as a result of which the cinchonidine is removed, and
(6) the liberated D(—)-3-benzoylthio-2-methylpropionic acid is won and purified.

If another mercapto-protective group than the benzoyl group is desired, this can be replaced by the other desired protective group in the final product in a way known per se. Examples of other protective groups are: acetyl, t-butyloxy and benzyloxy. These and other useful protective groups are also found in the above-mentioned Patent Application 7809121.

In addition to the process for preparing derivatives of proline the invention also relates to the proline derivatives themselves so prepared, as well as, of course, to the salts and esters derived therefrom. In this connection the ammonium salts and alcaline (earth) metal salts, such as the sodium-, potassium-, calcium- and magnesium salt, may be thought of. These can be prepared as described in the Dutch Patent Application, laid open for public inspection, No. 7701457. When esters are concerned the lower alkyl esters are primarily thought of.

The following preparation of 1-(3-mercapto-2-D-methylpropanoyl)-L-proline, which was prepared in three steps from D(—)-3-benzoylthio-2-methylpropionic acid in the way indicated in the formula sheet, serves to illustrate the invention.

*1°. Conversion into the acid chloride*

Under nitrogen atmosphere, 50 ml of thionyl chloride (purified by distillation from triphenylphosphite) were added to 33.6 g of D(—)-3-benzoylthio - 2 - methylpropionic acid ($[\alpha]_D$ = —45°; 1% in 96% ethanol). The mixture was set to stirr in a water bath of 40°C, in the course of which a homogeneous liquid mixture formed slowly. After approximately 6 hours the gas evolution had largely finished. By dissolving each time a small amount of the reaction mixture in methanol and chromatographing this solution on a TLC-plate the course of the reaction could be followed. When all the acid had disappeared, 25 ml of 1,2-dichloroethane were added.

The volatile components were removed from the mixture under reduced pressure, at a temperature between 40 and 45°C, after which D(—)-3-benzoylthio-2-methylpropionic acid chloride remained in an almost quantitative yield as an oil, being colourless to light yellow.

*2.° Reaction of the acid chloride with L-proline*

With stirring and at a temperature of 0°C, 6 g of the acid chloride obtained above were

added to a solution of 2.3 g of L-proline in 100 ml of acetone and 5 ml of dimethylaniline. A solution was obtained after 15 minutes' stirring. After additional stirring for 45 minutes at a temperature of 0°C, the mixture was poured out into 100 g of ice+water and 5 ml of concentrated HCl. The organic layer was separated and the water layer was extracted three times with 100 ml of methylene chloride. The methylene chloride extractions were combined with the organic layer to one organic phase, which was washed with 100 ml of ice-water + 4 ml of concentrated HCl and rewashed twice with 100 ml of ice-water. After drying over magnesium sulphate and evaporation of the organic solvents, 6.5 g = about 100% of crude 1-(3-benzoylthio-2-D-methylpropanoyl)-L-proline were obtained.

*3.° Debenzoylation*

The 6.5 g of 1-(3-benzoylthio-2-D-methylpropanoyl)-L-proline obtained under *2.°* were dissolved in 50 ml of methanol. Under nitrogen atmosphere, this solution was added to 0°C to a solution of 3.24 g of $NaOCH_3$ in 100 ml of methanol. The resulting solution was stirred for 1 hour at 0°C and then poured out on a mixture consisting of 100 g of melting ice and 30 ml of concentrated HCl.

After the mixture had been concentrated by evaporation under reduced pressure to a volume of approximately 100 ml, the residue was extracted with 6×50 ml of methylene chloride. The combined methylene chloride layers were extracted thrice with 50 ml-portions of a 5% solution of sodium bicarbonate in water. The assembled bicarbonate layers were acidified with concentrated HCl to a pH $\leqslant 1$, saturated with NaCl, and extracted with 6×50 ml of methylene chloride. After combining these six extractions the organic phase thus formed was concentrated by evaporation.

The result was 4.7 g (=84%) of crude 1-(3-mercapto - 2 - D - methylpropanoyl) - L - proline which appeared as an oil showing a light brown colour, and which solidified slowly.

Treatment of the crude product with hexane to which methylene chloride had been added, resulted in a crystalline product having a melting point of 100.9—102.9°C and an $[\alpha]_D = -127.4°$ (1% in 96% ethanol).

It is also possible to carry out the three steps without isolating the intermediate products. A preparation thus performed resulted as well in a yield of 84%, based on the starting compound D(—)-3-benzoylthio-2-methylpropionic acid.

As a later preparation in which, compared with the one referred to above, changes with respect to the solvent, reaction time and work-up process had been introduced, yielded better results, further particulars of that process are set out below.

The first step (conversion into the acid chloride) passed off identically. The second step (reaction of the acid chloride with L-proline) proceeded as follows:

With stirring and at a temperature between 0 and 5°C, the acid chloride was dosed out, in approximately one hour, to a solution of L-proline (5% excess) in dichloromethane and 2 equivalents of dimethylaniline, the solution being kept under nitrogen atmosphere. The coupling process completed (reaction time: over 4 hours), the reaction mixture was poured out on ice and diluted HCl. The organic layer was separated, washed twice with diluted HCl, in order to remove all the dimethylaniline and excess L-proline, and rewashed twice with water. After drying by means of magnesium sulphate the dichloromethane was distilled off. The yield of crude product — a thick oil — was between 95 and 100%.

The third (debenzoylating) step proceeded as follows:

The crude product obtained above was dissolved in methanol. Under nitrogen atmosphere, 2 equivalents of $NaOCH_3$ were added to that solution at a temperature of 0°C. The stirring was continued (for about 2 hours) until the reaction had finished.

The reaction mixture was neutralized with acetic acid to pH 5 and, subsequently, poured out on a mixture of ice and water. The methyl benzoate, which had likewise formed, was removed from the resulting mixture by extracting the mixture three times with toluene. The methanol was then distilled from the aqueous phase under reduced pressure, the reaction mixture was acidified with HCl to pH $\leqslant 1$, and salted out with NaCl.

The organic layer was separated. The aqueous layer was extracted (approximately five times) with dichloromethane until it gave a negative reaction with potassium nitroprusside. The combined dichloromethane extractions were added to the organic layer and this organic phase was dried by means of magnesium sulphate. After removal of the solvent there resulted an oil — yield between 90 and 95% — which had still to be crystallized. For that purpose the oil was dissolved in ethyl acetate, to which cyclohexane was then added slowly. As soon as any turbidity set in, some seed crystals were added to the solution and additional cyclohexane was dosed very slowly. The desired product crystallized out in an overall yield of 80—85% and had a melting point of 105—107°C and an $[\alpha]_D = -129.5°$ (1% in 96% ethanol).

Instead of starting from D(—)-benzoylthio-2-methylpropionic acid it is possible, of course, to utilise a D(—)-3-mercapto-2-methylpropionic acid in which the mercapto function is protected by a group different from benzoyl. As protective groups notably those referred to in our Dutch Patent Application No. 7809121, bearing the same priority date, may be considered.

The optical purity of the final product and of

the various intermediate products was determined by converting the products in ways known per se into the dimer of $\beta$-mercaptoisobutyric acid: $(-S-CH_2-CH(CH_3)-COOH)_2$. Using the HPLC it is possible to determine the various ratios of the relevant optical isomers of this compound quantitatively. Thus, it was found that during the process of converting S-benzoyl-$\beta$-mercaptoisobutyric acid into 1-(3-mercapto-2-D-methylpropanoyl)-L-proline the optical purity of the $\beta$-mercaptoisobutyric acid part underwent no change.

## Claims

1. Process for preparing proline derivatives, wherein a proline compound is reacted with a derivative of 3-mercapto-2-methylpropionic acid, which process comprises converting an optically active D(—)-3-mercapto-2-methylpropionic acid, of which the mercapto function is protected, into the corresponding acid chloride whereby the acid chloride during and after said conversion is maintained at a temperature of at most 45°C, and reacting the acid chloride with a L-proline compound according to formula I

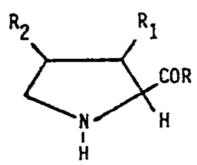

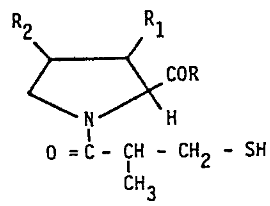

(I) (II)

in which R is a hydroxyl group, an amino group or a lower alkoxy group, and $R_1$ and $R_2$ represent independently a hydrogen atom, a hydroxyl group or a lower alkyl group, and liberating the protected mercapto function in order to obtain the desired proline derivative according to formula II in which R, $R_1$ and $R_2$ have the meanings indicated above and wherein the proline moiety still has the L-configuration and the acyl side chain the D-configuration.

2. Process according to claim 1, wherein R=OH and $R_1$=$R_2$=H.

## Patentansprüche

1. Verfahren zur Herstellung von Prolinderivaten, wobei man eine Prolinverbindung mit einem Derivat von 3-Mercapto-2-methyl-propionsäure reagieren lässt, dadurch gekennzeichnet, dass man eine optisch aktive D(—)-3-Mercapto-2-methylpropionsäure, deren Mercaptofunktion abgeschirmt ist, umsetzt in das entsprechende Säurechlorid, das während und nach der Umsetzung auf einer Temperatur von höchstens 45°C gehalten wird und dass man das Säurechlorid reagieren lässt mit einer L-Prolinverbindung nach der Formal I

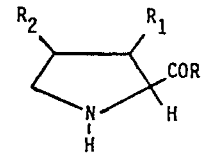

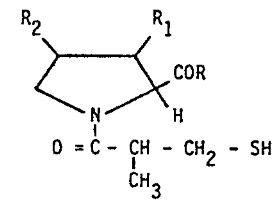

I II

in der R eine Hydroxylgruppe, eine Aminogruppe oder eine niedrigere Alkoxygruppe ist und $R_1$ und $R_2$ unabhängig voneinander ein Wasserstoffatom, eine Hydroxylgruppe oder eine niedrigere Alkylgruppe darstellen, und dass man die abgeschirmte Mercaptofunktion frei macht um das gewünschte Prolinderivat nach der Formal II zu erhalten, in der R, $R_1$ und $R_2$ die oben angegebenen Bedeutungen haben, und in der das Prolinteil noch die L-Konfiguration und die Acylseitenkette die D-Konfiguration hat.

2. Verfahren nach Anspruch 1, dadurch gekennzeichnet, dass R=OH und $R_1$=$R_2$=H.

## Revendications

1. Procédé de préparation de dérivés de la proline, dans lequel on fait réagir un composé de la proline avec un dérivé de l'acide 3-mercapto-2-méthylpropionique, qui consiste à transformer un acide D(—)-3-mercapto-2-méthylpropionique optiquement actif, dont la fonction mercapto est protégée, en le chlorure d'acide correspondant, le chlorure d'acide étant maintenu, pendant et après cette transformation, à une température maxima de 45°C, et à faire réagir le chlorure d'acide avec un composé de la L-proline répondant à la formule I

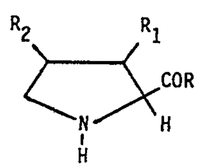

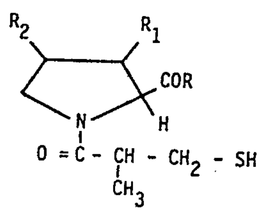

I II

dans laquelle R est un groupe hydroxy, un groupe amino, ou un groupe alkoxy inférieur, et $R_1$ et $R_2$ représentent indépendamment un atome d'hydrogène, un groupe hydroxy ou un groupe alkyle inférieur, et à libérer la fonction mercapto protégée pour obtenir le dérivé de la proline désiré répondant à la formule II, dans laquelle R, $R_1$ et $R_2$ ont les significations indiquées ci-dessus et dans laquelle la fraction proline a toujours la configuration L et la chaîne latérale acyle la configuration D.

2. Procédé suivant la revendication 1, caractérisé en ce que R=OH et $R_1$=$R_2$=H.

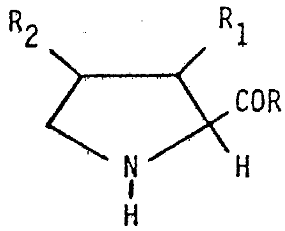

I

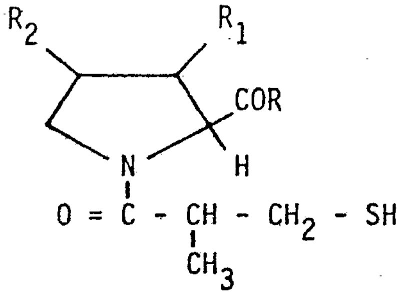

II